# EUROPEAN PATENT SPECIFICATION

(11) **EP 3 087 993 B1**
(45) Date of publication and mention of the grant of the patent: **04.07.2018**
(21) Application number: 16165670.7
(22) Date of filing: 15.04.2016
(51) Int. Cl.: A61K 38/08, A61K 38/39, A61K 38/48, A61K 9/00, A61P 21/02

(54) **PHARMACEUTICAL COMPOSITION COMPRISING ACETYL HEXAPEPTIDE-8 AND PENTAPEPTIDE-18 FOR TREATING ANAL FISSURES**
PHARMAZEUTISCHE ZUSAMMENSETZUNG ENTHALTEND ACETYL HEXAPEPTID-8 UND PENTAPEPTID-18 ZUR BEHANDLUNG VON ANALFISSUR
COMPOSITION PHARMACEUTIQUE COMPRENANT L'ACETYL HEXAPEPTIDE-8 ET LE PENTAPEPTIDE-18 POUR TRAITER LES FISSURES ANALES

(30) Priority: 17.04.2015 IT RM20150163
(43) Date of publication of application: 02.11.2016
(73) Proprietor: Uniderm Farmaceutici S.r.l., 00125 Rome (IT)
(72) Inventor: Chiozza, Giorgio, Rome (IT); Olmo, Sara, Rome (IT); Migliuolo, Moira, Rome (IT)
(74) Representative: Primiceri, Maria Vittoria

(56) References cited:
- WO-A1-2013/114410
- US-A- 5 674 205
- US-A1- 2005 214 327
- US-A1- 2009 068 219
- "A GMP PEPTIDE FOR COSMETIC APPLICATIONS A NEW SYNTHETIC COSMETIC INGREDIENT", , 1 June 2005 (2005-06-01), pages 1-7, XP055239218, Retrieved from the Internet: URL:http://www7a.biglobe.ne.jp/sysoap/pdf/ leuphasyl-TDS.pdf [retrieved on 2016-01-07]
- ANCA DRAGOMIRESCU ET AL: "The Efficiency and Safety of Leuphasyl-A Botox-Like Peptide", COSMETICS, vol. 1, no. 2, 1 January 2014 (2014-01-01) , pages 75-81, XP055238757, DOI: 10.3390/cosmetics1020075

## Description

### Technical field of the invention

The present invention relates to pharmaceutical compositions for treating anal fissures and other disorders associated with muscular hypertonia in the anal region.

### Prior art

Anal fissures (also known as anal rhagades) are ulcerations which are generally located at the posterior margin of the anus and, more rarely, may be located on the anterior margin.

Generically, the term *fissure* denotes a linear cutaneous ulceration involving both the epidermis and the more superficial layers of the dermis. The anus is one of the zones most affected by this type of ulceration because it is subjected to stretching and relaxation phenomena and its anterior and posterior margins are zones characterized by poor extensibility and reduced skin elasticity. When the skin breaks, a cut from which blood or serum losses occur is formed in the anal zone.

The fissures which form in this zone of the body, apart from being difficult to treat, have a tendency to recur.

The precise causes of anal fissures are as yet unknown, but it is thought that they may arise following excessive dilation of the anus resulting in laceration of the skin; one of the causes may, for example, be found in passing excessively bulky and hard faeces, as usually happens in individuals suffering from chronic constipation.

More recently, the formation of fissures has been attributed to inadequate blood supply to the skin caused by excessive muscle tone around the internal sphincter of the anal canal. The muscles, being excessively tense, would appear to cause a reduction in tissue vascularisation with consequent formation of ulcers. Anorectal manometry tests have demonstrated that the tone of the anal sphincter, measured as resting pressure, is very much higher in patients with anal fissures than in healthy individuals (Lin J-K. "Anal manometric studies in hemorrhoids and anal fissures" Dis Colon Rectum 1989; 32: 839-42.).

If the skin tear is not particularly extensive and defecation is regular, the wound can heal spontaneously within a few days without any further consequences; the problem does become recurrent, however, when repeated lacerations occur in the same zone, not allowng the wound to heal. When the laceration persists for at least six weeks, the fissure is classified as chronic.

A proper diet and good personal hygiene are necessary, first and foremost, for ensuring spontaneous healing of the lacerations. In order to accelerate and ensure complete healing, the anal sphincter must furthermore be relaxed; to date, this has been achieved by various therapeutic approaches, depending on the complexity of the case.

A less invasive approach involves applying creams and/or ointments for example containing nitrogen donors such as nitroglycerine which, by increasing the nitric oxide level in the tissues, bring about dose-dependent relaxation of the sphincter; this treatment has proven ineffective in the great majority of cases, due both to the many side-effects observed in patients and to the frequent recurrences which occur (Steele S.R., Madoff R.D. "Systematic review: the treatment of anal fissure." Aliment Pharmacol Ther. 2006 Jul 15;24(2):247-57.).

A more effective - but certainly also more invasive and often painful - therapeutic treatment is the injection of botulinum toxin with the aim of achieving relaxation of the anal sphincter muscle. This approach, apart from being costly, has a time limit due to the duration of the effectiveness of the botulinum toxin and the patient is obliged to repeat the treatment on a recurrent basis (*loc. cit*.).

The final therapeutic option is surgery which, while having moderately good prospects of success, is an invasive technique and risks causing the patient faecal incontinence which is not always temporary. Moreover, this approach cannot be used in patients with compromised continence, chronic diarrhoea or pre-existing lesions of the sphincter (*loc. cit*.).

A need therefore exists for a safe, non-invasive and non-surgical approach which is effective in obtaining relaxation of the anal sphincter muscles by reducing their hypertonia and promoting healing of the fissures. Argireline®, also known as acetyl hexapeptide-8 (Ac-Glu-Glu-Met-Gln-Arg-Arg-NH₂ -SEQ ID No. 1) is a peptide used in cosmetic compositions.

Leuphasyl®, also known as pentapeptide-18 (H-Tyr-D-Ala-Gly-Phe-Leu-OH, SEQ ID No. 2), is another peptide used in the field of cosmetics.

Dermonectin® (Vevy Codex 18.1926), also known as oligopeptide-5, is an active peptide (Lexicon Vevy Skin Care Instant Reports Year I, No. 1, June 1984) widely used in the field of cosmetics. It is characterised by the tetrapeptide with the sequence L-arginyl-glycyl-L-aspartyl-L-serine (Arg-Gly-Asp-Ser, SEQ ID No. 3).

### Brief description of the invention

Acetyl hexapeptide-8 and pentapeptide-18 have now unexpectedly been found to have a synergistic effect with oligopeptide-5 in reducing muscular hypertonia of the anal sphincter and promoting the fissure healing process.

The present invention accordingly provides a pharmaceutical composition comprising acetyl hexapeptide-8 and/or pentapeptide-18 in combination or not with oligopeptide-5, hereinafter also denoted Dermonectin®, for use in treating painful conditions of the anal region associated with muscular hypertonia in general and for treating anal fissures in particular.

The present invention also provides the method for preparing a pharmaceutical composition comprising at least one of acetyl hexapeptide-8 and pentapeptide-18 in combination with oligopeptide-5.

The invention further provides a kit comprising the composition with at least one of pentapeptide-18 and acetyl hexapeptide-8 and oligopeptide-5 and a reusable or single-use applicator containing effective doses of composition.

Further aspects will emerge from the detailed description of the invention.

### Brief description of the figures

Figure 1: Number of anal fissure patients subjected to manometric measurement;
Figure 2: Pre-treatment and post-treatment statistical results on the basis of mean resting pressure (MRP) measurements.

### Detailed description of the invention

The present invention relates to the use of a pharmaceutical composition comprising acetyl hexapeptide-8 and pentapeptide-18 in combination or not with oligopeptide-5 for treating painful conditions of the anal region associated with muscular hypertonia in general and for treating anal fissures in particular. According to the invention, the pharmaceutical composition is used in the treatment of acute, recurrent or chronic fissures.

The inventors have surprisingly found that there is a synergistic effect between the above-stated components capable of inducing an unexpected relaxation of the anal sphincter, thus promoting more rapid healing of the anal fissures.

All percentages mentioned below should be considered weight/weight percentages.

In the context of the present invention, acetyl hexapeptide-8 is used in concentrations which range from 0.00005% to 0.01%, preferably between 0.003% and 0.007%.

In a preferred embodiment, acetyl hexapeptide-8 is the commercial product Argireline®.

Pentapeptide-18 is used in concentrations which range from 0.000025% to 0.005%, preferably between 0.001% and 0.004%.

In a preferred embodiment, pentapeptide-18 is the commercial product Leuphasyl®.

In a preferred embodiment, a mixture of pentapeptide-18 and acetyl hexapeptide-8 is the commercial product Argirelox® and in a preferred embodiment said mixture is used in concentrations which range from 0.1% to 20%, preferably between 8% and 12%.

In the context of the present invention, oligopeptide-5 is the commercial product Dermonectin® and said product is used in concentrations which range from 0.01% to 10%, preferably between 2% and 6%. The active ingredient oligopeptide-5 present in the commercial product Dermonectin® may be used in concentrations which vary between 0.004% and 4%, preferably between 0.8 and 2.5%.

The composition according to the invention has a pH of between 3.5 and 6.5, preferably between 4.5 and 5.5.

According to a preferred embodiment, the composition takes the form of a gel, cream, emulsion, ointment, stick, spray, enema or suppository or any other form suitable for topical administration known to a person skilled in the art.

According to the invention, the composition may contain at least one pharmaceutically acceptable vehicle or excipient.

The composition according to the invention may be made in such a way as to allow release of the various active ingredients in a simultaneous, sequential or delayed manner.

According to the invention, the composition may be used in combination with other active ingredients known to be effective as muscle relaxants for simultaneous, sequential or delayed administration.

According to the invention, the composition may contain one or more pharmaceutically acceptable additives such as preservatives, antioxidants, acidifiers, chelating agents, perfumes, rheological additives, solubilisers or neutralising agents.

In particular, imidazolidinyl urea, benzoic acid or the salts or esters thereof, sorbic acid or the salts thereof, 4-hydroxybenzoic acid or the salts or esters thereof, phenoxyethanol, dimethylol dimethyl hydantoin (DMDM hydantoin), benzyl alcohol, isothiazolinones, dehydroacetic acid and sodium salt, parabens, vitamins (group A, C, E), lecithins, arginine, soda, citric acid, lactic acid, carbomer and derivatives thereof, hydroxyethylcellulose, xanthan gum, acrylate polymers or EDTA may advantageously be used.

The preservatives are preferably imidazolidinyl urea, potassium sorbate and phenoxyethanol.

The particularly preferred acidifier is citric acid.
According to the invention, the concentration of imidazolidinyl urea may vary between 0.1% and 1%, preferably between 0.25% and 0.40%.

According to the invention, the concentration of citric acid may vary between 0.1% and 1%, preferably between 0.20% and 0.40%.

According to the invention, the concentration of potassium sorbate may vary between 0.1% and 1%, preferably between 0.25% and 0.60%.

According to the invention, the concentration of phenoxyethanol may vary between 0.1% and 1%, preferably between 0.3% and 0.80%.

According to the invention, the composition may contain one or more humectant, hydrating, emollient, surfactant and emulsifying agents selected from among glycerin, butylene glycol, pentylene glycol, hydroxyphenyl propamidobenzoic acid, 4-tert.-butylcyclohexanol, polyoxyethylene stearate, glyceryl stearate, dimethicone, phenyl trimethicone, panthenol, synthetic or natural triglycerides, fatty acid esters, glycol stearate or the esters thereof, cetylstearyl alcohol, panthenol, plant extracts, bisabolol, aloe vera, vegetable oils, elastin or collagen.

The concentration of glycerin may vary between 1% and 10%, preferably between 3% and 8%.

The concentration of triglycerides, preferably of C10-18 triglycerides, may vary between 5% and 20%, preferably between 12% and 18%.

The concentration of C12-20 acid/PEG-8 ester may vary between 2% and 20%, preferably between 5% and 10%.

The concentration of acetylated glycol stearate may vary between 1% and 10%, preferably between 2% and 5%.

The concentration of 4-tert.-butylcyclohexanol may vary between 0.01% and 3%, preferably between 0.2% and 1.0%.

The concentration of cetylstearyl alcohol may vary between 0.5% and 10%, preferably between 1% and 3%.

The concentration of hydroxyphenyl propamidobenzoic acid may vary between 0.005% and 0.25%, preferably between 0.03% and 0.07%.

The concentration of glyceryl stearate may vary between 0.1% and 5%, preferably between 0.5% and 1.8%.

The concentration of the mixture of polyoxyethylene (10) isocetyl stearate and polyoxyethylene (10) isostearyl stearate may vary between 0.1% and 2%, preferably between 0.15% and 1%.

The concentration of dimethicone may vary between 0.1% and 10%, preferably between 0.3% and 3%.

The concentration of the mixture of glyceryl stearate and PEG-100 stearate may vary between 0.5% and 10%, preferably between 0.5% and 3%.

The solvent used to bring the final weight of the composition up to 100% is selected from among water, alcohols, glycols, oils selected from those normally used in the field of cosmetics and pharmaceuticals.

According to a preferred embodiment, the composition of the invention comprises, apart from acetyl hexapeptide-8 and/or pentapeptide-18 in combination with oligopeptide-5, at least one of hydroxyphenyl propamidobenzoic acid, butylene glycol, pentylene glycol, 4-tert.-butylcyclohexanol and corresponding mixtures.

In a preferred embodiment, 4-tert.-butylcyclohexanol is the commercial product Symsitive®.

In a preferred embodiment, a mixture of hydroxyphenyl propamidobenzoic acid, butylene glycol and pentylene glycol is the commercial product Symcalmin®

Another aspect of the invention relates to the method for preparing the composition in cream form which is characterized by the following stages:
introducing the raw materials into a melter and turboemulsifier according to the procedures and quantities specified by the formula.

The raw materials placed in the melter melt at a temperature which may vary from 65 to 80°C, preferably between 70°C and 75°C. The raw materials placed in the turboemulsifier are heated to a temperature which may vary from 60 to 80°C, preferably between 65°C and 75°C.

The emulsion is formed by combining the previously melted raw materials placed in the melter with the previously heated raw materials present in the turboemulsifier.

The emulsion is then cooled down until a temperature which may vary from 45 to 60°C, preferably between 50 and 55°C, is reached.

At this point, the "glycerin", Symcalmin (hydroxyphenyl propamidobenzoic acid, butylene glycol and pentylene glycol) and the preservative system may be introduced into the emulsion. Subsequently, once a temperature which may vary from 30 to 45°C, preferably between 35 and 40°C, has been reached, the active raw materials may be introduced into the emulsion: Pentapeptide-18, acetyl hexapeptide-8 and oligopeptide-5.

The emulsion is then acidified until the desired pH is obtained.

The composition according to the invention may be used alone or in combination with other active ingredients or treatment methods for anal fissures.

Another aspect of the invention consists of a kit comprising the composition with at least one of pentapeptide-18 and acetyl hexapeptide-8 and oligopeptide-5 and a reusable or single-use applicator containing effective doses of composition, alternatively single- or multi-use premeasured packages containing the composition may be provided.

The inventors have unexpectedly found that just 60 minutes after first application, the composition provided by the invention was capable of reducing the tone of the anal sphincter, measured as resting pressure, in all treated individuals. A more detailed study has shown how said myorelaxant effect is attributable to a synergistic action between acetyl hexapeptide-8 and pentapeptide-18 with Dermonectin. In those patients treated with the composition of the invention, not only was a reduction in the muscle tone of the sphincter observed, but more rapid healing of the fissure was also obtained.

The following examples are to be regarded as being merely illustrative and not limiting the scope of the invention.

### EXAMPLES

### Characterization of Dermonectin®

A fibronectin derivative, having the characteristics that it retains the biologically active sites, has a low molecular weight and is readily assimilated by the skin. Fibronectin's ability to bond to cells is attributed in the literature to the presence of the tetrapeptide: L-arginyl-glycyl-L-aspartyl-L-serine (SEQ ID No. 3). Dermonectin is characterised by the presence of this tetrapeptide.

Dry residue hydrolysis of Dermonectin reveals the characteristic amino acid profile shown in Table 1.

**Table 1**

| | | | |
|---|---|---|---|
| Alanine | 6-9% | Arginine | 3-5% |
| Aspartic acid | 8-10% | Glutamic acid | 6-9% |
| Phenylalanine | 4-6% | Glycine | 4-6% |
| Isoleucine | absent | Histidine | 2-4% |
| Lysine | 5-7% | Serine | 3-5% |
| Proline | 1-3% | Valine | 4-6% |
| Threonine | 2-4% | Leucine | 6-8% |
| Tyrosine | 1-3% | Hydroxyproline | absent |

Appearance at 20°C: clear liquid
Colour: straw yellow
pH (10%, 18°C): 6.4 ± 0.2
Density (18°C): 1.0566 ± 0.002
Soluble in aqueous and aqueous-alcoholic systems Total nitrogen in dry residue: 13%
Non-hydrolyzed protein content: 85%
Moisture: max. 5%
Ash: 7%
Heavy metals: < 2 ppm (Pb, Hg, As absent)

UV-Vis in distilled water solution with a concentration of 0.1521% weight/volume (g/ml 100). Operating conditions:
- wavelength range: 300-210 nm;
- scanning speed: 30 nm/min;
- paper speed: 20 mm/min;
- E: 0-0.5;
- O.P.: cm 1;
- Blank solution: distilled water.

The graph shows a peak with an absorption maximum at 257 nm.

### Amino acid analysis after acid hydrolysis (Table 2)

(Methodology: high-performance liquid chromatography of AMINO ACIDS (AFTER ACID HYDROLYSIS) **NEOTRON** method: AMINOAC-tot 2014 Rev.2 HPLC-FL
INSTRUMENT: HPLC WITH FLUORESCENCE DETECTOR - results stated by weight (g/100g)

**Table 2 (Source: Uniderm analytical laboratory)**

| | |
|---|---|
| Total aspartic acid(*) | 0.930 |
| Total glutamic acid(**) | 0.688 |
| Alanine | 0.814 |
| Arginine | 0.430 |
| Phenylalanine | 0.455 |
| Glycine | 0.412 |
| Hydroxyproline | <LQ |
| Isoleucine | <LQ |
| Histidine | 0.269 |
| Leucine | 0.711 |
| Lysine | 0.621 |
| Proline | 0.291 |
| Serine | 0.334 |
| Tyrosine | 0.146 |
| Threonine | 0.285 |
| Valine | 0.537 |

| | |
|---|---|
| (*) including asparagine (**) including glutamine LQ = quantitation limit (equal to 0.1) | |

### Preparation of the emulsion

Introduce the raw materials into the melter and turboemulsifier according to the procedures and quantities specified by the formula.

The raw materials placed in the melter melt at a temperature of between 70°C and 75°C. The raw materials placed in the turboemulsifier must be heated to a temperature of between 65°C and 75°C.

The emulsion is formed by combining the previously melted raw materials placed in the melter with the previously heated raw materials present in the turboemulsifier.

The emulsion is then cooled down until a temperature of between 50 and 55°C is reached.

At this point, the "glycerin", Symcalmin (hydroxyphenyl propamidobenzoic acid, butylene glycol and pentylene glycol) and the preservative system may be introduced into the emulsion. Subsequently, once a temperature of between 35 and 40°C has been reached, the active raw materials may be introduced into the emulsion: Pentapeptide-18, acetyl hexapeptide-8 and oligopeptide-5.

The emulsion is then acidified until the preferred pH is obtained.

The phases of the process are shown by way of example in the following diagram:

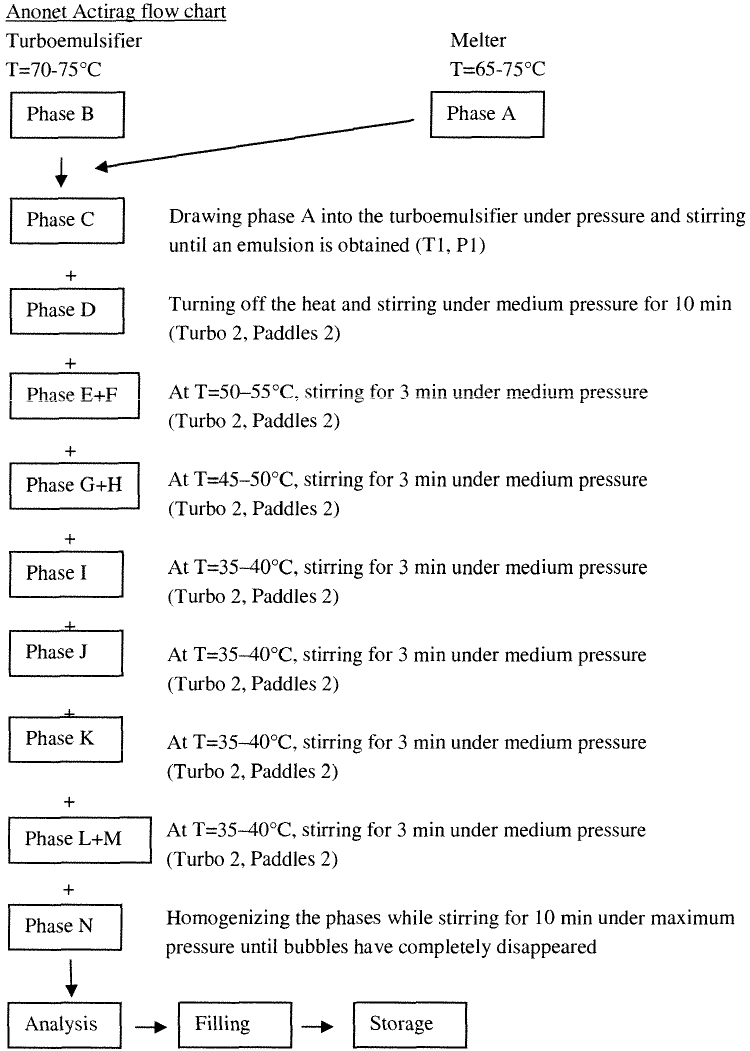

### COMPONENTS OF THE PHASES:

| ***PHASES*** | ***COMPONENTS*** |
|---|---|
| Phase A | Cetacene (acetylated glycol stearate) |
| | Arlacel 165PAMV (glyceryl stearate PEG-100 stearate) |
| | Acesil 350 (dimethicone) |
| | Xalifin-15 (C12-C20 acid PEG-8 ester) |
| | Cetylstearyl Alcohol 50/50 (cetearyl alcohol) |
| | Isoxal-h (polyoxyethylene 10) |
| | Nesatol (C10-C18 triglycerides) |
| | Cutina GMS SE (glyceryl stearate SE) |
| | Symsitive (pentylene F glycol, 4-tert.-butylcyclohexanol) |
| Phase B | Water (aqua) |
| Phase E | Glycerin nat veg fu 99.5% (glycerin) |
| | Protectol PE (phenoxyethanol) |
| Phase G | Potassium sorbate |
| | Preservative G (imidazolidinyl urea) |
| | Water (aqua) |
| Phase I | Symcalmin (butylene glycol pentylene glycol hydroxyphenyl propamidobenzoic acid) |
| Phase J | Dermonectin (oligopeptide-5) |
| Phase K | Argirelox (acetyl hexapeptide-8, peptide-18) |
| Phase L | Citric acid |
| | Water (aqua) |

### Example of a composition containing oligopeptide-5, acetyl hexapeptide-8 and pentapeptide-18.

| **INCI name** | **% by weight in formula** |
|---|---|
| WATER | 56.4220 |
| C10-18 TRIGLYCERIDES | 15.0000 |
| C12-20 ACID/PEG-8 ESTER | 8.5000 |
| GLYCERIN | 6.0000 |
| ACETYLATED GLYCOL STEARATE | 2.5000 |
| OLIGOPEPTIDE-5 | 1.9500 |
| GLYCERYL STEARATE | 1.7500 |
| PENTYLENE GLYCOL | 1.6750 |
| CETEARYL ALCOHOL | 1.5000 |
| 4-TERT.-BUTYLCYCLOHEXANOL | 0.8000 |
| PEG-100 STEARATE | 0.7500 |
| PHENOXYETHANOL | 0.6000 |
| DIMETHICONE | 0.5000 |
| BUTYLENE GLYCOL | 0.4750 |
| POTASSIUM SORBATE | 0.4000 |
| ISOCETETH-10 STEARATE | 0.3125 |
| IMIDAZOLIDINYL UREA | 0.3000 |
| CITRIC ACID | 0.2700 |
| ISOSTEARETH-10 STEARATE | 0.1875 |
| CAPRYLYL GLYCOL | 0.0500 |
| HYDROXYPHENYL PROPAMIDOBENZOIC ACID | 0.0500 |
| ACETYL HEXAPEPTIDE-8 | 0.0050 |
| PENTAPEPTIDE-18 | 0.0030 |

### Preliminary study

The primary and secondary aims of the study were respectively to evaluate variations in manometric parameters and any adverse events.

Patients considered eligible for inclusion in this preliminary study were those of either sex, aged between 18 and 70, with an anal fissure, whether acute or chronic. The patients were evaluated clinically (medical history and objective examination) and were subjected to proctoscopy and anorectal manometry. An evaluation of anal pain was furthermore obtained using a ten-point visual analogical scale (VAS).

Patients suffering from Crohn's disease, ulcerative colitis, TBC, HIV, malignant colorectal disease, anal incontinence and patients with a history of pelvic radiotherapy and previous anorectal surgery were excluded.

After the clinical evaluation, all the patients meeting the inclusion criteria were subjected to a single administration, by topical endo/perianal anal application of the preparation shown in Example 1 and contained in suitable containers bearing no logo. The quantity of preparation applied was standardised at 3 grams.

One hour after application, a further manometric investigation was carried out.

### Anorectal manometry

Anorectal manometry was performed using a 4.8 mm diameter silicone catheter provided with eight lumens and four radial openings located 5 cm from the tip and perfused with twice-distilled water by means of a low-compliance pump at a rate of 0.75 mL per minute and flow of 1.2 kg/cm² (Menfis Biomedica, Bologna, Italy). The catheter was connected to a personal computer via a transducer. The catheter, appropriately lubricated, was introduced into the rectum with the patient in left lateral decubitus position. The continuous pull-through method was used, carried out by means of an automatic retractor calibrated at a rate of 0.5 cm/s, so permitting an evaluation with regard to anal canal length (ACL), high pressure zone (HPZ) and mean resting pressure (MRP). The mean queeze pressure (MSP) was furthermore obtained by the stationary pull-through method.

### Statistical method

Statistical analysis was performed using the Stat Graph-Pad Prism® 5 application (San Diego, California, USA). The values are stated as mean ± standard deviation. The data were compared by means of the Wilcoxon test or t-test as indicated. Values of p of less than 0.05 were considered significant.

### Results

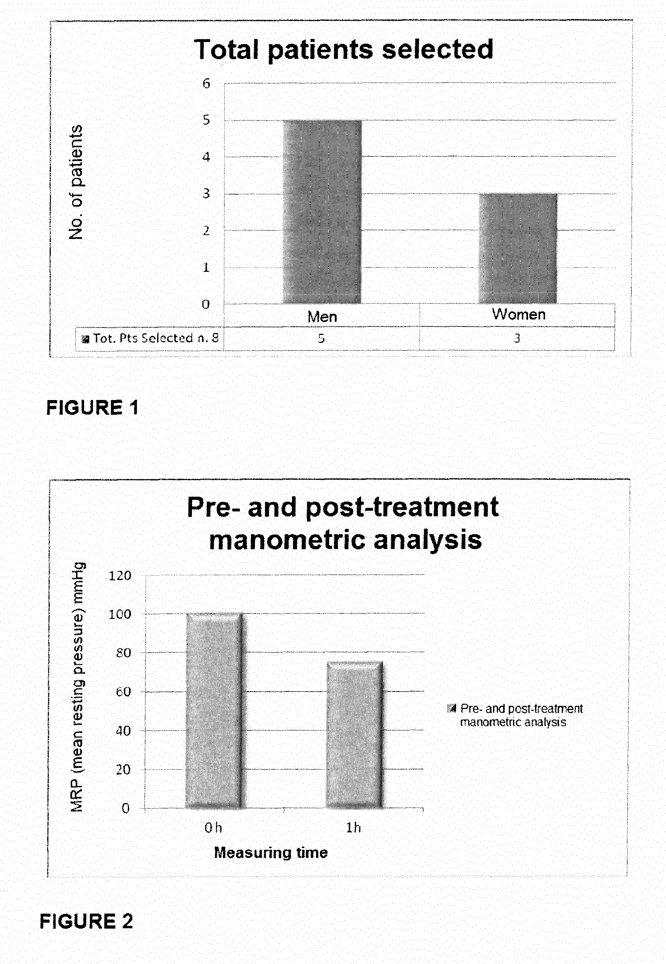

Of the patients observed, 8 met the inclusion criteria (Figure 1);
These patients [5 (62.5%) men and 3 (37.5%) women] had an average age of 39 (range 22-55). The main symptom was anal pain with onset during defecation and of variable duration, with a prevalence of 88.8% (8/9) and a mean VAS score of 8.

Anal bleeding upon defecation and chronic constipation were respectively reported by 50% (4/8) and 25% (2/8) of the patients.

Analysis of the manometric data pre- and post-application of the preparation, while it did not reveal any appreciable differences for the parameters ACL, HPZ and MSP, did in contrast reveal a statistically significant reduction in the parameter MRP [100.01 ± 12.2 vs. 75.08 ± 10.3 mmHg (*p*< 0.0001; paired t-test)] (Figure 2).

### Conclusions

It can be stated that application of the investigated preparation brought about a statistically significant myorelaxant effect (reduction of mean resting pressure, MRP) which is universally considered to be a necessary factor for achieving clinical healing of an anal fissure.

### Second study

The aim of study is to evaluate the variation in clinical parameters (healing, sphincter tone, pain), manometric parameters and any adverse events.

Patients considered eligible for inclusion in this study were those of either sex, aged between 18 and 70, with an anal fissure, whether acute or chronic. The patients were evaluated clinically (medical history and objective examination) and were subjected to proctoscopy and anorectal manometry. An evaluation of anal pain was furthermore obtained using a ten-point visual analogical scale (VAS).

Patients suffering from Crohn's disease, ulcerative colitis, TBC, HIV, malignant colorectal disease, anal incontinence, and patients with a history of pelvic radiotherapy and previous anorectal surgery were excluded.

After clinical evaluation, all the patients meeting the inclusion criteria were subjected to twice daily administration, by topical endo/perianal anal application, of the preparation supplied to them. The quantity of preparation applied was standardized at 3 grams.

Using a randomized and controlled examination procedure, the patients were divided into three groups and respectively received:
**Group I:** a salve containing solely Argirelox (acetyl hexapeptide-8, pentapeptide-18);
**Group II:** a salve containing Argirelox (acetyl hexapeptide-8, pentapeptide-18) together with Dermonectin (oligopeptide-5)
**Group III:** a salve containing exclusively a placebo

Each patient, irrespective of the group to which he/she belonged, received the salve in appropriate "unlabelled" containers in the quantity required for one month of treatment.

Randomization, generated by computer, was performed using the variable block method with a size of 4 to 6. On the basis of the resultant randomization list, paramedical staff who were not involved in analysing the data assigned each patient to one of the groups. The manometric evaluation prior to treatment and at 2 and 4 weeks was considered to be an integral part of the study together with the clinical/proctological evaluation and the evaluation of pain using the visual analogical scale as stated above.

### Anorectal manometry

Anorectal manometry was performed using a 4.8 mm diameter silicone catheter provided with eight lumens and four radial openings located 5 cm from the tip and perfused with twice-distilled water by means of a low-compliance pump at a rate of 0.75 mL per minute and flow of 1.2 kg/cm² (Menfis Biomedica, Bologna, Italy). The catheter was connected to a personal computer via a transducer. The catheter, appropriately lubricated, was introduced into the rectum with the patient in left lateral decubitus position. The method used was the continuous pull-through method, carried out by means of an automatic retractor calibrated at a rate of 0.5 cm/s, which permitted an evaluation with regard to anal canal length (ACL), high pressure zone (HPZ) and mean resting pressure (MRP). The mean squeeze pressure (MSP) was furthermore obtained by the stationary pull-through method.

### Statistical method

Statistical analysis was performed using the Stat Graph-Pad Prism® 5 application (San Diego, California, USA). The values are stated as mean ± standard deviation. The data were compared by means of the Wilcoxon test or t-test as indicated. Values of *p* of less than 0.05 were considered significant.

This study revealed the synergistic effect of the active ingredients (Argirelox and Dermonectin), demonstrating a greater myorelaxant effect in Group II in comparison with the individual active ingredient (Argirelox) in Group I, so giving rise to a better result in resolving the disorder.

## Claims

1. A composition comprising at least one of acetyl hexapeptide-8 (Ac-Glu-Glu-Met-Gln-Arg-Arg-NH2) and pentapeptide-18 (H-Tyr-D-Ala-Gly-Phe-Leu-OH) in combination with oligopeptide-5 (Arg-Gly-Asp-Ser) for use in treating disorders associated with muscular hypertonia of the anal region.

2. A composition for use according to claim 1, wherein the disorders are selected from acute, recurrent or chronic anal fissures.

3. A composition for use according to either one of claims 1-2, wherein acetyl hexapeptide-8 is Argireline® and pentapeptide-18 is Leuphasyl®.

4. A composition for use according to either one of claims 1-2, wherein a mixture of acetyl hexapeptide-8 and pentapeptide-18 is Argirelox®.

5. A composition for use according to any one of claims 1-4, further comprising at least one of hydroxyphenyl propamidobenzoic acid, butylene glycol, pentylene glycol, 4-tert.-butylcyclohexanol and corresponding mixtures.

6. A composition for use according to any one of claims 1-5, wherein acetyl hexapeptide-8 is present in a concentration which varies between 0.00005% and 0.01%, preferably between 0.003% and 0.007%.

7. A composition for use according to any one of claims 1-6, wherein pentapeptide-18 is present in a concentration which varies between 0.000025% and 0.005%, preferably between 0.001% and 0.004%

8. A composition for use according to any one of claims 1-7, wherein Dermonectin is present in a concentration which varies between 0.01% and 10% and preferably between 2% and 6%, or oligopeptide-5 is present in a concentration which varies between 0.004% and 4% and preferably between 0.8% and 2.5%

9. A composition for use according to any one of claims 1-8, wherein the pH varies between 3.5 and 6.5, preferably between 4.5 and 5.5.

10. A composition for use according to any one of claims 1-9, which further comprises at least one pharmaceutically acceptable excipient or vehicle.

11. The composition for use according to any one of claims 1-10, wherein said composition takes the form of a gel, cream, emulsion, salve, ointment, stick, spray, enema or suppository.

12. The composition for use according to any one of claims 1-11, in association with at least one active ingredient known for treating fissures and/or a method for treating anal fissures.

13. Acetyl Hexapeptide-8 in combination with pentapeptide-18 for use in treating disorders associated with muscular hypertonia of the anal region, preferably in treating anal fissures.

14. A kit comprising the composition according to any one of claims 1-12 in predefined dosage units, a reusable or single-use applicator containing effective doses of composition and instructions for use.

## Patentansprüche

1. Zusammensetzung, umfassend mindestens eines von Acetylhexapeptid-8
(Ac-Glu-Glu-Met-Gln-Arg-Arg-NH2)
und Pentapeptid-18
(H-Tyr-D-Ala-Gly-Phe-Leu-OH)
in Kombination mit Oligopeptid-5
(Arg-Gly-Asp-Ser)
zur Verwendung in der Behandlung von Erkrankungen, die mit Muskelhypertonie der Analregion assoziiert sind.

2. Zusammensetzung zur Verwendung nach Anspruch 1, wobei die Erkrankungen ausgewählt sind aus akuten, wiederkehrenden oder chronischen Analfissuren.

3. Zusammensetzung zur Verwendung nach einem der Ansprüche 1 bis 2, wobei Acetylhexapeptid-8 Argireline® und Pentapeptid-18 Leuphasyl® ist.

4. Zusammensetzung zur Verwendung nach einem der Ansprüche 1-2, wobei eine Mischung aus Acetylhexapeptid-8 und Pentapeptid-18 Argirelox® ist.

5. Zusammensetzung zur Verwendung nach einem der Ansprüche 1-4, weiterhin umfassend mindestens eines von Hydroxyphenylpropamidobenzoesäure, Butylenglykol, Pentylenglykol, 4-tert.-Butylcyclohexanol und entsprechende Mischungen.

6. Zusammensetzung zur Verwendung nach einem der Ansprüche 1 bis 5, wobei Acetylhexapeptid-8 in einer Konzentration vorliegt, die zwischen 0.00005% und 0.01%, vorzugsweise zwischen 0.003% und 0.007%, variiert.

7. Zusammensetzung zur Verwendung nach einem der Ansprüche 1 bis 6, wobei Pentapeptid-18 in einer Konzentration vorliegt, die zwischen 0.000025% und 0.005%, vorzugsweise zwischen 0.001% und 0.004%, variiert.

8. Zusammensetzung zur Verwendung nach einem der Ansprüche 1-7, wobei Dermonectin in einer Konzentration vorliegt, die zwischen 0.01% und 10% und vorzugsweise zwischen 2% und 6% variiert, oder Oligopeptid-5 in einer Konzentration vorliegt, die zwischen 0.004% und 4% und vorzugsweise zwischen 0.8% und 2,5% variiert.

9. Zusammensetzung zur Verwendung nach einem der Ansprüche 1-8, wobei der pH-Wert zwischen 3.5 und 6.5, vorzugsweise zwischen 4.5 und 5.5, variiert.

10. Zusammensetzung zur Verwendung nach einem der Ansprüche 1-9, die zudem mindestens einen pharmazeutisch annehmbaren Hilfsstoff oder Trägersubstanz umfasst.

11. Zusammensetzung zur Verwendung nach einem der Ansprüche 1-10, wobei die besagte Zusammensetzung die Form eines Gels, einer Creme, einer Emulsion, einer Salbe, eines Balsams, eines Stifts, eines Sprays, eines Einlaufs oder eines Suppositoriums aufweist.

12. Zusammensetzung zur Verwendung nach einem der Ansprüche 1-11 in Verbindung mit mindestens einem zur Behandlung von Fissuren bekannten Wirkstoff und/oder einem Verfahren zur Behandlung von Analfissuren.

13. Acetylhexapeptid-8 in Kombination mit Pentapeptid-18 zur Verwendung bei der Behandlung von Erkrankungen, die mit Muskelhypertonie der Analregion assoziiert sind, vorzugsweise bei der Behandlung von Analfissuren.

14. Kit, umfassend die Zusammensetzung nach einem der Ansprüche 1-12 in vordefinierten Dosierungseinheiten, einen wiederverwendbaren oder einen einmal verwendbaren Applikator beinhaltend eine wirksame Dosierungen der Zusammensetzung, und eine Gebrauchsanweisung.

## Revendications

1. Une composition comprenant au moins un d'acetyl hexapeptide-8 (Ac-Glu-Glu-Met-Gln-Arg-Arg-NH2) et pentapeptide-18 (H-Tyr-D-Ala-Gly-Phe-Leu-OH), en combinaison avec un oligopeptide-5 (Arg-Gly-Asp-Ser) pour l'utilisation dans un traitement de troubles liés à l'hypertonie musculaire du domaine anal.

2. Une composition pour l'utilisation selon la revendication 1, dans laquelle les troubles sont choisis entre fissures anales aigues, récurrentes ou chroniques.

3. Une composition pour l'utilisation selon l'une des revendications 1 à 2, dans laquelle l'acetyl hexapeptide-8 est Argireline® et le pentapeptide-18 est Leuphasyl®.

4. Une composition pour l'utilisation selon l'une des revendications 1 à 2, dans laquelle un mélange de l'acetyl hexapeptide-8 et du pentapepdid-18 est Argirelox®.

5. Une composition pour l'utilisation selon l'une quelconque des revendications 1 à 4, comprenant en outre au moins l'acide hydroxyphenylpropamidobenzoique, butylène glycol, pentylène glycol, 4-tert.-butylcyclohexanol et de mélanges correspondants.

6. Une composition pour l'utilisation selon l'une quelconque des revendications 1 à 5, dans laquelle l'acetyl hexapeptide-8 est présent dans une concentration qui varie entre 0.00005% et 0.01%, préférablement entre 0.003% et 0.007%.

7. Une composition pour l'utilisation selon l'une quelconque des revendications 1 à 6, dans laquelle le pentapeptid-18 est présent dans une concentration qui varie entre 0.000025% et 0.005%, préférablement entre 0.001% et 0.004%.

8. Une composition pour l'utilisation selon l'une quelconque des revendications 1 à 7, dans laquelle Dermonectin est présent dans une concentration qui varie entre 0.01% et 10% et préférablement entre 2% et 6% ou l'ogliopeptide-5 est présent dans une concentration qui varie 0.004% et 4%, préférablement entre 0.8% et 2.5%.

9. Une composition pour l'utilisation selon l'une quelconque des revendications 1 à 8, dans laquelle le pH varie entre 3.5 et 6.5, préférablement entre 4.5 et 5.5.

10. Une composition pour l'utilisation selon l'une quelconque des revendications 1 à 9, qui comprend en outre au moins un excipient pharmaceutiquement acceptable ou un véhicule pharmaceutiquement acceptable.

11. Une composition pour l'utilisation selon l'une quelconque des revendications 1 à 10, dans laquelle la dite composition prend la forme d'un gel, crème, émulsion, baume, onguent, bâton, spray, lavement, ou suppositoire.

12. Une composition pour l'utilisation selon l'une quelconque des revendications 1 à 11, en association avec au moins un ingrédient actif connu pour le traitement de fissures et /ou un procédé de traitement de fissures anales.

13. Acetyl hexapeptide-8 en combinaison avec pentapeptide-18 pour l'utilisation dans le traitement de troubles liés à l'hypertonie musculaire du domaine anal, préférablement dans le traitement de fissures anales.

14. Un kit comprenant la composition selon l'une quelconque des revendications 1 à 12 dans des unités posologiques prédéfinies, un applicateur réutilisable ou à utilisation unique contenant des doses efficaces de composition et des instructions d'utilisation.


### SEQUENCE LISTING

<110> Uniderm Farmaceutici Srl
<120> Pharmaceutical compositions for treating anal fissures
<130> EPI-13514
<150> RM2015A000163
   <151> 2015-04-17
<160> 3
<170> PatentIn version 3.5
<210> 1
   <211> 6
   <212> PRT
   <213> Homo sapiens
<400> 1
   Glu Glu Met Gln Arg Arg
   1               5
<210> 2
   <211> 5
   <212> PRT
   <213> Homo sapiens
<400> 2
   Tyr Ala Gly Phe Leu
   1               5
<210> 3
   <211> 4
   <212> PRT
   <213> Homo sapiens
<400> 3
   Arg Gly Asp Ser
   1